# EUROPEAN PATENT SPECIFICATION

(11) **EP 4 301 421 B1**
(45) Date of publication and mention of the grant of the patent: **10.07.2024**
(21) Application number: 22712545.7
(22) Date of filing: 28.02.2022
(51) Int. Cl.: A61L 2/10, H05B 47/115

(54) **DYNAMIC DISINFECTING VIRTUAL ROOM DIVIDERS BY UV LIGHT**
DYNAMISCHE DESINFEKTION VON VIRTUELLEN RAUMTEILERN DURCH UV-LICHT
DÉSINFECTION DYNAMIQUE DE DIVISES-PIÈCES VIRTUELS PAR LUMIÈRE UV

(30) Priority: 03.03.2021 US 202163155929 P; 01.04.2021 EP 21166498
(43) Date of publication of application: 10.01.2024
(73) Proprietor: Signify Holding B.V., 5656 AE Eindhoven (NL)
(72) Inventor: WENDT, Matthias, 5656 AE Eindhoven (NL); DEIXLER, Peter, 5656 AE Eindhoven (NL)
(74) Representative: Verweij, Petronella Daniëlle
(86) International application number: PCT/EP2022/054922
(87) International publication number: WO 2022/184609

(56) References cited:
- CN-A- 112 190 727
- CN-U- 212 631 291
- US-A1- 2019 224 350
- US-A1- 2019 298 869

## Description

### FIELD OF THE INVENTION

The present invention relates to a disinfecting device and system for disinfecting indoor spaces with UV, IR or visible light. The invention further relates to a method for disinfecting an indoor space using the aforementioned disinfecting device.

### BACKGROUND OF THE INVENTION

In all indoor spaces with human occupants there is a risk of pathogens (such as a virus) spreading between individuals. A virus can be spread by physical contact between individuals either directly (e.g. by means of a handshake) or indirectly by individuals touching e.g. a same door handle, light switch or touch screen. Moreover, pathogens may spread through the indoor air as large (e.g. larger than 100 µm) or small (e.g. smaller than 100 µm) particles. Large particles such as droplets of saliva or respiratory fluid can be expelled from an infected individual during coughing, sneezing or even talking. If not wearing a mask the large droplets may travel ballistically and infect another individual by impacting directly or otherwise coming into contact with the the eyes, nostrils or mouth of the other individual. Furthermore, small aerosol particles of saliva or respiratory fluid can also be expelled from an infected individual. Depending on the size of the aerosol particles containing the pathogen such particles can linger in the indoor air for long periods of time, from a few seconds to several hours. The small lingering aerosol particles can later infect an individual by deposition on the eyes or via inhalation.

Existing solutions for decreasing the risk of pathogen spread in indoor spaces include social distancing, use of protective gear such as gloves and disinfecting agents being employed by the occupants so as to mitigate the spread of pathogens via physical contact. The amount of small aerosol particles with pathogens in the air is reduced by increased ventilation rates of the indoor air or by use of upper air UV disinfection systems which operate by irradiating the upper air of the indoor space or the air circulating in the HVAC system with hazardous germicidal UV radiation which disinfects the aerosols. To mitigate the potentially ballistic spread of large droplets from infected individuals sneezing, coughing or talking face covering masks are used such that the number and reach of expelled droplets is reduced.

US 2019/224350 A1 discloses a lighting device with lighting elements emitting disinfecting light. The lighting device comprises components like a local controller and a lens. The lens is configured to diffuse the emitted light. The emitted light may thereby be distributed in different ways using different distribution profiles.

A problem with existing solutions is that the spread of pathogens in indoor spaces is still not as efficient as is desired with infections still occurring due to e.g. individuals not following recommendations regarding use of face covering masks, gloves or disinfecting agent. Another problem lies in the pathogen reaching an individual via a lingering aerosol or ballistic large particles before being treated by any upper air or HVAC disinfection system.

### SUMMARY OF THE INVENTION

In view of the shortcomings of the existing solutions there is a need for an improved disenfection device, disinfection system and method for using the disinfecting device to disinfect an indoor space and mitigate spread of pathogens. It is an object of the present invention to overcome these shortcomings and to provide such an disenfection device, disinfection system and method for using the disinfecting device.

The invention relates to a disinfecting device according to the appended claim 1 and to a method for controlling a disinfecting device according to the appended claim 14. Further features are disclosed in the dependent claims.

According to a first aspect of the invention, this and other objects are achieved by a disinfecting device according to the appended claim 1 and comprising an elongated light bar configured to be mounted to a surface of an indoor space. The light bar includes at least one light source (such as a UV, IR or 405 nm light source) configured to output a disinfecting light, and at least one optical element, configured to shape the light emitted by the light source(s), such that the light bar is configured to emit light in an optical plane extending from a longitudinal axis of the light bar. The light bar further comprises a slant angle controller configured to when the disinfecting device is mounted to a surface, control a slant angle between the optical plane and the surface to which the disinfecting device is mounted to.

With optical plane it is meant the center plane of the light distribution extending from the light bar. The light extending in the optical plane when the disinfecting device (light bar) is mounted to a surface may be referred to as a light curtain.

In aspects, the light extending in the optical plane may substantially be confined to the optical plane, with a slight tolerance of e.g. at most 2 centimeter (perpendicular) offset from the optical plane, preferably at most 1 centimeter (perpendicular) offset from the optical plane.

The slant angle between a normal of the surface and the optical plane is the angle between the optical plane and a normal of the surface to which the disinfection device is mounted. That is, with a slant angle of 0 degrees the optical plane extends perpendicular to the surface. The longitudinal axis of the light bar splits the surface into two sides, a first side and a second side. For a slant angle between 0 degrees and +90 degrees the optical plane extends from the longitudinal axis and forms an acute angle with the first side of the longitudinal axis and an obtuse angle with the second side. For a slant angle of +90 degrees the optical plane is parallel to the surface and extends along the surface on the first side of the longitudinal axis. For a slant angle between 0 degrees and -90 degrees the optical plane forms an acute angle with the second side of the longitudinal axis and for a slant angle of -90 degrees the optical plane extends in parallel with the surface and along the second side. The surface may be a ceiling or a floor of the indoor space. The surface may be a wall of the indoor space. When installed to a surface the disinfecting light of the disinfecting device will extend from the light bar in the optical plane so as to form a disinfecting light curtain.

The present invention is at least partially based on the understanding that by projecting disinfecting light in an optical plane which delimits the indoor space the audibility and visibility of the indoor space is maintained while both aerosol and large particles with pathogens are disinfected so as to decrease the risk of pathogen spread from one side of the optical plane to the other side. The disinfecting device may in terms of pathogen spread isolate an open indoor space in the latitudinal direction (i.e. the direction perpendicular to the longitudinal extent of the optical plane) without obscuring the audibility and visibility properties of the space. The disinfecting device may more effectively stop pathogen spread via aerosol particles and large ballistic particles as it does not rely on the air circulation to bring the pathogen particles to e.g. a disinfecting device provided in an HVAC system serving the indoor space or an upper air UV disinfecting device.

Moreover, as the optical plane is controlled by the slant angle controller the emitted disinfecting light may be controlled so as to facilitate more efficient or safer disinfection of the indoor space. For example, by controlling the slant angle of a ceiling or floor mounted disinfection unit away from 0 degrees the disinfecting light ray length in the air is increased which increases the probability of the emitted disinfecting photons encountering and deactivating a pathogen before being absorbed by a surface of the indoor space. For example the disinfecting light is UV light which is hardly absorbed by air and the UV light retains its germicidal function until it reaches a surface and is, at least partially, absorbed by the surface. Accordingly, the efficiency of a UV light curtain is enhanced. For instance, a lower intensity UV light source or UV radiation which has a higher maximum recommended dosage for humans but also features a lower germicidal efficiency may be used to obtain the same disinfection efficiency as non-slanted UV light curtains with e.g. higher intensity UV light. By controlling the slant angle to maximize the UV ray length of the UV curtain, the disinfecting device may maximally contribute to the air disinfection of the indoor space.

In implementations, the slant angle controller controls the slant angle in an optimal slant angle range, wherein the range of optimal slant angles is between ± 25 and ± 65 degrees, such as ± 45. Accordingly, for each slant angle in the range of optimal slant angles the UV ray propagation length in air is increased which enhances the disinfection efficiency and provides the benefits as mentioned in the above.

Additionally, slant angle control allows for reduced power consumption, ease of use, increased system life-time and minimizing the disinfecting light exposure experienced by occupants of the indoor space while maximizing the emitted disinfecting dose (e.g. UV dose) from the disinfecting device.

The disinfecting properties of the disinfecting device may be controlled by adjusting the slant angle of the light curtain. The control may be manually operated by a user manipulating a switch, button or touch interface of a user device which is in communication with the control unit of the disinfecting device. The control of the slant angle may be automatic and respond to an input signal from a sensor or scheduled to adjust the slant angle with respect to a time of day. For example, the disinfecting device may be scheduled to project a disinfecting light curtain with a larger slant angle at nights and/or on the weekends when the occupant activity is assumed to be low. Accordingly, the efficiency and disinfecting properties of the disinfecting light curtain may be controlled so as to best suit the indoor space or a current and/or future context of the indoor space.

Moreover, in aspects, said elongated light bar may alternatively be a light bar, or a light module. Said light module may e.g. comprise an elongated shape, but may alternatively comprise a circular shape, or be a point source of light capable of rendering said light curtain according to the invention with said at least one optical element.

In some implementations the light source(s) is at least one of an ultraviolet (UV) light source, an infrared (IR) light source and a visible light source. Said light source(s) may e.g. be configured to emit visible light comprising light with a maximum (peak) intensity at a wavelength between 400 nm and 410 nm, such as 405 nm. UV light (such as UV-C) has been a proven technology for disinfecting air, water and instruments for over a century. Niels Finsen was awarded the Nobel Prize for Medicine in 1903 for being the first to use "light therapy" to treat disease with direct disinfection of skin. The drawback of UV-C light is that it is not friendly to humans or certain surface materials. That is, as soon as the UV-C light is off bacteria can grow again. Hence, recently companies have been looking for disinfection lighting alternatives which create an unfriendly space to bacteria while being friendly to humans. For example, UV-A technology may be applied, which may be considered eye-safe for continuous disinfection in the presence of humans for up to 8 hours a day, unlike UV-C. Moreover, disinfection devices operating entirely in the visible spectrum, especially in the near UV spectrum of 400 nm to 410 nm have been demonstrated to effectively disinfect pathogens without employing UV light. For example, Indigo-Clean by Kenall uses 405 nm visible purple light in operating rooms which is claimed to be safe for both patients and caregivers and to kill any bacteria that survives routine cleaning.

IR light may also feature a disinfectant effect. An advantage of using IR light as disinfection light is that it works better than UV light on certain surfaces and for certain pathogens. For example, it is well known that NIR disinfection (750-950 nm) achieves an 80% to 99.9% (or 2-3 log) reduction of the iron-dependent and some other types of bacteria and fungi. Furthermore, the absorption spectrum of each pathogen shows some distinct absorption peaks. Consequently, very specific IR or UV wavelengths may be selected so as to `excite' specific bond types in the specific molecules in the targeted pathogens. The disinfectant lighting may comprise visible light in addition to the UV and IR light. Different UV, IR and visible light sources may be combined so as achieve tailored deactivation of a certain selection of pathogens.

In some embodiments the slant angle controller is configured to control the positioning of the optical element with respect to the light source(s).

Slant angle adjustability of the optical plane may be enabled by changing the positioning (i.e. moving and/or reorienting) at least one of the optical element and the light source(s). For example, the optical element is oriented or moved with respect to the light source(s) or vice versa, which results in a slant angle adjustment of the projected disinfecting light curtain. The optical element and the light source(s) may both move simultaneously. The movement may be enabled by at least one actuator of the light bar, controlled by the slant angle controller.

In some implementations the disinfecting device comprises an actuating unit and the slant angle controller is configured to control the actuating unit. The actuating unit in turn being configured to steer the orientation of the light bar with respect to the surface when said disinfecting device is mounted to a surface.

For example, the light bar may be configured to output disinfecting light in a fixed optical plane relative to the light bar wherein the slant angle controller controls an actuating unit which is configured to steer the orientation of the light bar to control the resulting slant angle. The actuating unit provides e.g. at least rotation around the longitudinal axis.

In some implementations the light bar is configured to emit disinfecting light in two optical planes, wherein the slant angle of the two optical planes is different. Wherein the slant angle controller is configured to selectively control the light bar to selectively emit disinfecting light in one of said two optical planes so as to select one slant angle or another.

For example, this may be enabled by the slant angle controller merely activating and/or deactivating a different light source(s) of the disinfecting device wherein the different light sources are oriented with respect to the optical element so as to project a light curtain with a different slant angle. In some embodiments a plurality of light sources is provided with a same or individual optical elements, by the control unit selecting which light sources to activate and/or deactivate the slant angle (or slant angle width as is discussed in the below) of the resulting disinfecting light curtain may be adjusted accordingly. In this way, the disinfecting device may comprise no moveable parts while still enabling steerability of the slant angle by selectively controlling one or more of the light sources. To enable steerability of the slant angle the optical element may be an LCD element or the light bar may comprise an LCD element in addition to the optical element. Wherein the LCD element is configured to actively shape the distribution of the emitted light around the optical plane or control the slant angle of the optical plane.

In some implementations the slant angle controller is configured to communicate with a context awareness sensor and control the slant angle of the optical plane based on a context of the indoor space being detected by the context awareness sensor.

The context awareness sensor may be an integrated part of the disinfecting device and/or provided separately (e.g. communicating wirelessly with the slant angle controller). More than one presence sensor may be used cooperatively so as to e.g. determine the occupant context in different parts of the indoor space. The slant angle controller of a ceiling or floor mounted disinfecting device may be configured to steer the optical plane so as to approach a slant of approximately 90 degrees when the occupant context indicates occupant presence. In this way, the light curtain requires a smaller floor area as it minimizes the area in which an occupant breaks the curtain. The context awareness sensor may for example sense the presence, level of activity and number of occupants in the indoor space with the slant angle controller controlling slant angle with respect to the occupant context. To this end, the context awareness sensor may e.g. be a presence sensor.

In one example, the context awareness sensor detects that a person is sleeping in the indoor space (e.g. by establishing the presence of one occupant and a low level of activity for that occupant). In response, the slant angle controller may steer the slant angle such that the light curtain is closer to eyes of the occupant compared to if the occupant was awake.

Further, the context awareness sensor may be configured to detect the position/activity of an occupant relative the disinfecting device and wherein the slant angle controller is configured to control the slant angle of the optical plane based on the position of the occupant.

The position of an occupant may comprise the distance between the occupant and the disinfecting device or the positioning of the occupant in the indoor space. The context awareness sensor may comprise a distance sensor configured to measure the distance between an occupant and the disinfecting device. The slant angle controller may be configured to steer the slant angle such that the disinfecting curtain is at least a predetermined distance away from an occupant and/or the eyes of an occupant. For example, the slant angle controller may determine the slant angle providing the highest disinfecting efficiency while still being at least a predetermined distance away from each occupant in the indoor space. The predetermined distance may be at least 20 cm, such as 30 cm or 50 cm. The predetermined distance from an occupant and/or the occupant's eyes may further be modified by the sensed activity of the occupants. For example, if an occupant or a group of occupants is performing an activity associated with heavy breathing (such as singing or exercising) the slant angle controller may control the optical plane to be closer to the occupant(s) performing the activity associated with heavy breathing.

The light source(s) may comprises a plurality of light elements distributed along the elongated light bar and the slant angle controller is configured to activate a subset of the light elements to control an extent of the light curtain along the longitudinal axis.

Accordingly, the longitudinal width of the light curtain may be further controlled by the slant angle controller. By increasing/reducing the longitudinal width of the emitted curtain the efficiency of the disinfecting device is enhanced since only a necessary curtain width may be enabled. A plurality of light sources may be arranged side by side along the longitudinal axis of the light bar with a common or separated optical element(s) to enable the slant angle controller to selectively control all or a subset of the light sources in order to control the width of the emitted disinfecting light curtain.

In some embodiments the disinfecting device comprises a visible spectrum light source configured to project visible light, the projected visible light indicating the position of the optical plane.

The visible light source may be provided in the light bar alongside the light sources and feature little or no disinfecting properties. The visible light may be referred to as a visible light curtain. For example, the visible light curtain may be 405 nm blue light which features some germicidal properties that contribute to the germicidal properties of the disinfecting light curtain. Moreover, any visible light may be used to indicate the position of the disinfecting light curtain to enable occupants to easily realize the position, presence and/or slant angle of the disinfecting light curtain. Accordingly, occupants may be discouraged to cross a visible light indicator so as to avoid being exposed to disinfecting light (which may be UV or IR light invisible for the naked eye). Occupants may e.g. choose to converse or move about the indoor space in a manner wherein a visible light curtain at all times separates any two occupants (which indicates that a disinfecting curtain also separates the occupants and mitigates the spread of pathogens). The visible light may be a linear light projection onto a surface, e.g. the floor, which indicates where on that same surface the disinfecting light is incident. The visible light source may be an integrated part of the disinfecting device or provided externally. The visible light source may be a laser or a projector.

In some implementations the light bar is further configured to emit an enveloping light surrounding the disinfecting light emitted in the optical plane and having a different spectrum.

The enveloping light envelopes or surrounds the optical plane in at least the direction of the normal of the optical plane (the slant angle direction). The envelope light may be UV or IR or 400 to 410 nm light having a spectra different from UV light of the optical plane. For example, the envelope light is UV-B or IR while the UV light of the optical is UV-C. Accordingly, disinfection is facilitated as the disinfection properties of two light spectra may be combined. For example, a less effective (in terms of germicidal effect) but also less hazardous UV or IR light spectra may be used for the enveloping light while a more effective but also more hazardous light spectra (e.g. UV-C) may be used for the disinfecting light projected in the enveloped optical plane. Accordingly, the more hazardous UV radiation will be confined to a smaller space. The slant angle controller may be configured to control both the edge light and the optical plane such that the edge light envelops the optical plane of the disinfecting light curtain.

As mentioned, in some implementations, the light source(s) may be at least one of: an ultraviolet (UV) light source, an infrared (IR) light source and a visible light source. In some implementations, the UV light source is configured to output UV light with a wavelength in a range of 100 nm to 400 nm, or preferably in a range of 190 nm to 280 nm. In some implementations, the IR light outputted by an IR light source is in a wavelength range of 700 nm to 1200 nm and preferably in a wavelength range of 700 to 1000 nm.

In some implementations the slant angle controller is configured to also control the intensity of the disinfecting light outputted by the light source(s).

The intensity includes the on/off state of the disinfecting light (represented by an intensity of zero and non-zero respectively). Accordingly, the intensity of the disinfecting light may be increased or decreased by the controller so as to facilitate efficient disinfection of the indoor space. For example, the intensity of the disinfecting light may be increased during times when the indoor space is known to be busy. Additionally, the light bar of the disinfecting device may be configured to output disinfecting light of different spectra with the controller being further configured to selectively control which spectra the emitted disinfecting light features. In combination with a context awareness sensor the controller may be adapted to turn off the disinfecting light in response to an occupant being detected in the vicinity (e.g. within a predetermined minimum distance from a disinfecting light curtain). This control may be configured with eye safety and/or light exposure safety regulations and the disinfecting light spectra of the light source(s) in mind. For example, with a UV-B light source an occupant is allowed to come closer to the UV curtain or spend a longer period of time inside the curtain prior to the controller changing the slant angle and/or the intensity of the UV curtain compared to when the UV light source is a UV-C light source.

In some implementations the slant angle controller controls the spectrum of the emitted disinfection light based on the slant angle. As the slant angle is steered away from 0 degrees (especially for a disinfection device mounted to a ceiling or a floor) the disinfection light is more likely to reach an occupant's eyes, either directly or by the disinfection light being reflected of surfaces of the indoor space due to an increased angle of incidence. Also, as the slant angle is steered away from 0 degrees the disinfection curtain may be projected over a larger area of the indoor space and reach a larger number of occupants.

To this end, the slant angle controller may continuously or discontinuously change the spectrum or the type of disinfection light emitted for different slant angles. For example, for each absolute slant angle exceeding a predetermined threshold angle the slant angle controller may control the light bar to emit disinfection light associated with higher recommended human exposure limits. For each absolute slant angle below the predetermined threshold angle the slant angle controller may control the light bar to emit disinfection light associated with a lower recommended human exposure level. The predetermined threshold angle may be any angle in a range from 0 degrees to 90 degrees, such as 30, 45 or 60 degrees.

In an exemplary embodiment, the slant angle controller controls the light bar to emit less harmful UV-B light (or IR light or 405 nm visible light) when the slant angle is between 90 and 45 degrees while the slant angle controller controls the light bar to emit more harmful UV-C when the slant angle is between 45 and 0 degrees (and similarly for the negative slant angles). More than two spectrums may be combined and the slant angle ranges for the respective light spectrum provided in the above are merely exemplary.

Moreover, by modulating the intensity of the outputted disinfection light of each spectrum a gradual transition from one spectrum to another may be enabled by allowing a combination of two spectrums to be emitted for some slant angles. For example, the slant angle controller controls the light bar to emit 405 nm visible disinfection light when the slant angle is between 90 and 45 degrees, UV-B light when the slant angle is between 60 and 30 degrees and UV-C light when the slant angle is between 45 and 0 degrees (with the equivalent light spectrums and angles being defined for the negative slant angles). Wherein, e.g. the intensity of the 405 nm light may gradually decrease and the UV-B light intensity gradually increases when the slant angle moves from 45 degrees to 30 degrees.

The disinfection device may comprise or be configured to be in communication with a reflection sensor configured to detect the light emitted from the light source(s) of the light bar after being reflected from the indoor space, and wherein the slant angle of the optical plane is controlled in response to a deviation exceeding a predetermined threshold value in the sensed reflected light being detected by the reflection sensor.

With a reflection sensor, the state of an occupant breaching the disinfecting curtain may be determined. In response to such a state being detected the slant angle controller may e.g. rapidly change the slant angle so as to bring the occupant out of the disinfecting curtain so as to decrease the exposure of the occupant. Implementing a reflection sensor that is integrated and/or external to the disinfecting device enables detecting when a disinfecting curtain is breached, e.g. by an occupant passing by. For example, the time series deviations of the reflections may be analyzed to infer the presence of a person (similar to structured light sensors). Moreover, the intensity of the disinfecting light may be decreased in response to a deviation exceeding a predetermined threshold value in the sensed reflected disinfecting light being detected by the disinfecting reflection sensor. The placement of the reflection sensor may be such that it detects reflected light from the vicinity of an area in which the eyes or head of occupants are often present. For example, the reflection sensor may detect the reflected light from the head portion of a bed. If variation exceeding the predetermined threshold value is detected the slant angle controller may establish that the bed is empty or that an occupant of the bed is sleeping with the slant angle controller adjusting the slant angle accordingly.

In some implementations the disinfecting device is integrated into a ceiling or wall luminaire. The disinfecting device may be integrated with the support structure which holds the indoor ceiling. In some implementations the disinfecting device is configured to be mounted to a track lighting rail and be moved along the track lighting rail.

According to a second aspect of the invention there is provided a disinfecting system, according to the appended claim 13, and comprising a plurality of disinfecting devices and a central control unit, wherein the central control unit is configured to control the slant angle controller of each disinfecting device.

The central control unit may convey slant angle instructions to each disinfecting device, the slant angle instructions may indicate a slant angle for each disinfecting device which enables each occupant to be isolated in a personal space delimited by at least one disinfecting light curtain. Moreover, the controller of each disinfecting device may be configured to control the intensity and/or the spectral distribution of the emitted disinfecting light. Accordingly, the central control unit may selectively activate and control disinfecting curtains when situations with risk of pathogen spread are detected, e.g. two occupants standing close to each other and talking. The disinfecting system may comprise one or more context awareness sensors conveying information to the central control unit which in turn controls the slant angle controller of each disinfecting device. The disinfecting system may form a grid with disinfecting units being oriented so as to provide different optical planes extending from longitudinal and latitudinal axis in the indoor space. Accordingly, with a disinfection system comprising a plurality of disinfecting devices only the necessary disinfecting devices may be put to use, e.g. those which may separate two occupants. In some, implementations the whole ceiling or floor of an indoor space is covered by distributed disinfecting devices so as to provide disinfecting curtains in an adaptive manner which maximizes disinfection efficiency and efficiency with respect to power usage and system lifetime while adhering to exposure regulations (e.g. recommended UV exposure levels if the light source(s) comprise a UV light source).

According to a third aspect of the invention there is provided a method for controlling a disinfecting device, according to the appended claim 14, wherein said disinfecting device is mounted to a surface of an indoor space such that the disinfecting light emitted by the light bar forms a disinfecting curtain in the optical plane. The method comprising the steps of emitting a disinfecting curtain and controlling the slant angle of the disinfecting curtain with respect to a normal of the surface.

The steps of the method may be repeated. For example, as the number of detected occupants changes or the position of an occupant changes the slant angle may be controlled in substantially real time so as to e.g. always be at least a predetermined distance away from the occupant.

Further, the method may comprise the steps of determining the position of a first occupant and a second occupant in the indoor space relative to the disinfecting device; and controlling the slant angle of the disinfecting curtain so as to separate said first occupant from said second occupant with the disinfecting curtain.

Accordingly, the occupants of the indoor space may be separated by a disinfecting curtain which mitigates the spread of pathogens from one occupant to another occupant. Also, only activating the light curtain(s) when it is enabled (via slant angle adjustments) to be situated between two occupants enhances the life time of the light source (as it may be deactivated for a large period of time), decreases power consumption and decreases potential degradation due to light-exposure (e.g. UV light exposure) of objects in the indoor space. The activity of the two occupants may be taken into consideration when controlling the slant angle. For example, in a hospital room the context awareness sensor may sense that two occupants are present, one occupant laying or sleeping in a bed (a patient) and a second occupant standing next to the bed (a nurse or doctor). There are potentially a wide range of slant angles which could be employed to separate the occupants and the slant angle controller may chose to move the curtain closer to the standing occupant (which is assumed to breath more heavily) and further from to the sleeping occupant (which is assumed to breath not as heavily). If the context awareness sensor detects that the sleeping patient starts coughing or talking the slant angle may be adjusted by the slant angle controller to bring the light curtain closer to the patient occupant.

The invention according to the second and third aspects features the same or equivalent embodiments and benefits as the invention according to the first. Further, any structural features described in relation to a device or unit, may have the corresponding steps in a method. It is noted that the invention relates to all possible combinations of features recited in the claims, e.g. the method may comprise the step of controlling the extent of the disinfecting curtain along the longitudinal axis by selectively controlling a subset of the disinfecting light elements.

In an aspect, the invention provides: a disinfecting device comprising: a light module configured to be mounted to a surface of an indoor space, said light module comprising: at least one light source, configured to emit disinfecting light for disinfecting pathogens, and at least one optical element, configured to shape disinfecting light emitted by the light source(s), such that the light bar is configured to emit disinfecting light in an optical plane extending from a longitudinal axis of the light bar; and a slant angle controller, configured to, when the disinfecting device is mounted to a surface, control a slant angle between the optical plane and a normal of the surface. Thereby, advantages and/or embodiments applying to the disinfection device according to the invention may mutatis mutandis apply to the disinfection device comprising a light module according to this present aspect of the invention.

### BRIEF DESCRIPTION OF THE DRAWINGS

This and other aspects of the present invention will now be described in more detail, with reference to the appended drawings showing embodiment(s) of the invention.
Fig. 1a depicts a disinfecting device according some embodiments of the invention.
Fig. 1b depicts a disinfecting device with up-conversion phosphorous according to some embodiments of the invention.
Fig. 2 depicts a disinfecting device mounted to a surface of an indoor space.
Fig. 3. depicts a disinfecting device mounted to a surface of an indoor space with emitted disinfecting curtains of different slant angles.
Fig. 4a depicts a disinfecting device mounted to a surface of an indoor space with emitted disinfecting curtains of different slant angles and slant angle widths.
Fig. 4b depicts a disinfecting device mounted to a surface of an indoor space emitting an outer and an inner enveloping disinfecting curtains.
Fig. 5 depicts a disinfection system comprising a plurality of disinfecting devices according to some implementations.
Fig. 6 is a flow chart of a method according some implementations.

### DETAILED DESCRIPTION

The present invention will now be described more fully hereinafter with reference to the accompanying drawings, in which currently preferred embodiments of the invention are shown. This invention may, however, be embodied in many different forms and should not be construed as limited to the embodiments set forth herein; rather, these embodiments are provided for thoroughness and completeness, and fully convey the scope of the invention to the skilled person.

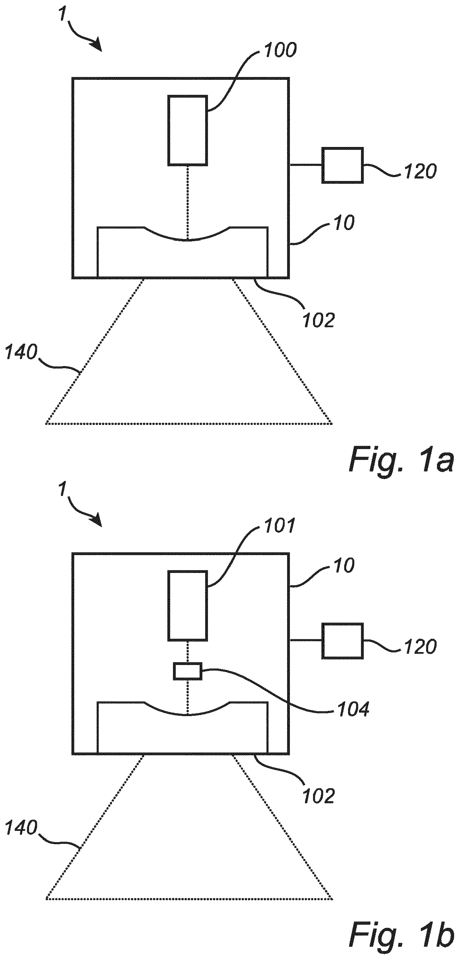

Fig. 1a depicts a disinfecting device 1 according to some implementations. A disinfecting device 1 or a system of disinfecting units may be integrated to a surface of an indoor space, e.g. the disinfecting device(s) 1 may be integrated to a ceiling. The disinfecting device comprises a light bar 10 wherein the light bar comprises a light source(s) 100 and an optical element 102. The light bar is configured to emit disinfecting light in an optical plane 140 extending from a longitudinal axis. The disinfecting light emitted by the light bar 10 may diverge in along the longitudinal axis as the disinfecting light propagates away from the light bar 10 as shown in Fig. 1a. Alternatively, the disinfecting light emitted by the light bar 10 may feature an approximately constant extension along the longitudinal axis as it propagates away from the light bar 10. Especially useful is the disinfecting device(s) 1 in indoor spaces in which where many people meet, e.g. in conference auditoriums, school rooms, meeting rooms, hospital rooms, gyms, fitness centres, museums, churches, serenity rooms, offices, elderly homes visitor rooms, operation centers, cinemas, theatres, concert halls, restaurants, and shopping centers.

A slant angle controller 120 is provided to control the slant angle of the optical plane 140 of the disinfecting light. To enable control of the slant angle (which tilts the disinfecting curtain 140 in or out of the plane of Fig. 1a) of the disinfecting light curtain 140 the light bar 10 of the disinfecting device 1 may comprise an actuated optical element 102 controlled by the slant angle controller 120. For example, the position and/or orientation of the optical element 102 may be adjusted by an actuator, wherein the actuator is controller by the slant angle controller 120, such that the slant angle in which the disinfecting light curtain 140 is outputted by the optical element 102 is adjusted accordingly. Additionally or alternatively, the position and/or orientation of the light source 100 may be adjusted by an actuator, wherein the actuator is controlled by the slant angle controller 120, such that the slant angle of the disinfecting light curtain 140 outputted by the optical element 102 is adjusted accordingly. The light source 100 and/or the optical element 102 may both be fixed or individually actuated with the slant angle controller controlling the slant angle being by an actuator unit which controls the position and/or orientation of the light bar 10 with respect to a surface when the disinfecting device 100 is mounted to the surface.

Some implementations enable controllability of the slant angle by the light bar 10 being configured to emit disinfecting light in two optical planes 140, wherein the slant angle of the two optical planes is different and the slant angle controller 120 is configured to selectively control the light bar 10 to selectively emit disinfecting light in one of said planes. The different optical planes extending from a respective longitudinal axis wherein the longitudinal axis are parallel to each other and wherein the optical axis may be superimposed or displaced from each other. For example, the light bar 10 may be realized without moving parts as a plurality of light sources 100 may be provided in the light bar 10 with the light sources 100 being oriented with respect to the optical element to provide an individual slant angle when activated. For instance, the optical plane 140 of the disinfecting light projected by the optical element 102 from a first light source 100 is slanted with a first slant angle when the first light source 100 is activated and the optical plane of the disinfecting light projected by the optical element 102 is slanted with a second slant angle when a second light source is activated. The first slant angle being greater than the second slant angle and the slant angle of light bar 10 being adjustable by the slant angle controller 120 controlling which light source is activated.

In some implementations the light source 100 is a disinfecting laser light source 100. The light source may be any collimated disinfecting light source 100 and different light source types which enable to generating a sufficiently concentrated light and hence enables a projection of collimated planar disinfecting light may be used. The disinfecting light outputted from the light source 100 may be distributed as a beam of some finite beamwidth and the outputted disinfecting light is transmitted through and shaped by the optical element 102 which concentrates the disinfecting light into a linear projection extending in the optical plane 140, forming a disinfecting curtain. In other implementations a plurality of light sources 100 are arranged along the longitudinal axis in order to generate an elongated beam or a linear distribution of individual beams with the optical element shaping the elongated distribution into light extending from the longitudinal axis in the optical plane 140. Examples of optical elements 102 suited for concentrating disinfecting light into a linear projection comprises a cylindrical lens and/or a diffractive element.

In one example, disinfecting device of Fig. 1a is controlled by the slant angle controller to emit disinfecting light in an optical plane forming a slant angle of 5 degrees relative a normal of the surface onto which it is mounted. That is, the disinfecting light curtain of Fig. 1a may be slanted so as to project out of the plane of Fig. 1a.

The light source 100 may be configured to output a narrowband disinfecting spectrum or any spectral range of UV light. For example, the light bar 10 and UV light source 100 may be configured to output UV light one or more of the Ultra Deep UV range of 100 to 190 nm, the Deep UV light range of 190 to 220 nm, the UV-C range of 220 to 280 nm, the UV-B range of 280 to 315 nm and/or the UV-A light of 315 to 400 nm. A narrowband UV light source may be configured output UV light with a narrow wavelength distribution around e.g. 254 nm or 255 nm. The light source 100 may be configured to output IR light such as light in the range of 700 nm - 1 mm, such as 750 - 900 nm. The light source may emit visible disinfecting light such as with a peak intensity at between 400 and 410 nm, for example a narrow band visible light with peak intensity at 403, 408 or 405 nm.

With reference to Fig. 1b there is depicted an exemplary disinfecting device 1 utilizing a light bar 10 with up-conversion phosphorus 104 for generating UV light. In some implementations, the UV light of the light bar 10 is generated by illuminating up-conversion phosphorus 104 with a visible pump light 101, e.g. blue light emitted by a blue LED 101. A collimated UV light source may be enabled by pumping the above mentioned up-conversion phosphorous 104 with visible laser light, e.g. with a blue laser 101. It is noted that other combinations of visible pump light and frequency up-converting luminescing materials may be applied to achieve a UV light source according to embodiments of the present invention.

In some implementations, the light bar 10 may be configured such that a portion of the visible pump light from the visible light source 101 is deliberately transmitted through the conversion phosphorous 104 or up-converting material to become superimposed with the UV light curtain extending in the optical plane 140. The visible pump light may thereby indicate the presence, current position (e.g. current slant angle, intensity) of the UV light curtain. For example, the visible light may be reflected of other surfaces in the indoor space so as to make an occupant aware of the position of the UV light curtain by perceiving the reflected visible light. If the disinfecting device 1 is mounted to a ceiling an occupant may perceive a visible light linear projection across the floor and extending up along the walls in a certain slant angle towards the disinfecting device 1 which indicates, at least approximately, the extension of the UV light curtain (being superimposed with the visible light).

In some implementations, the visible light may be generated separately from the disinfecting light and be configured to follow the slant angle of the disinfecting light curtain so as to indicate a current position of the disinfecting light curtain. For example, a visible light source of the disinfecting device 1 is calibrated so as to coincide with the disinfecting light curtain at least where the disinfecting curtain is first reflected (such as surface of the indoor space opposite the surface onto which the disinfecting device is mounted and in the propagation direction of the disinfecting light). For an occupant to perceive the position of a UV or IR curtain it suffices to indicate one or more positions where the UV or IR curtain is incident on a surface. To follow the disinfecting light curtain the visible light source may be actuated and calibrated with information pertaining to, the placement of the disinfecting device 1 in the indoor space, the placement of the visible light source in the indoor space and/or the dimensions of the indoor space.

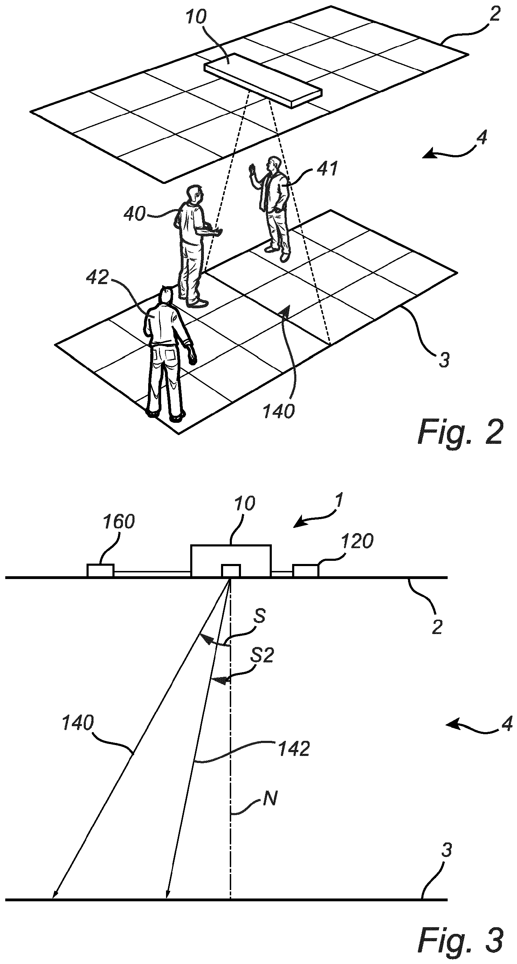

Fig. 2 depicts a disinfecting device 1 mounted to a surface 2 (such as a ceiling or a floor) of an indoor space 4. The disinfecting device 1 of Fig. 2 is mounted to a ceiling 2 with the disinfecting curtain extending towards the floor 3. The disinfecting device 1 emits a disinfecting light curtain 140 extending in an optical plane 140 so as to e.g. separate a first occupant 40 from a second occupant 41 of the indoor space 4. Potential pathogens spreading ballistically or by being suspended in the air may be disinfected as they pass the disinfecting light curtain 140. The slant angle controller of the disinfecting device 1 may control the slant angle of the disinfecting curtain so as to separate the two occupants 40, 41 even if one or both of the occupants 40, 41 moves in the indoor space. A context awareness sensor with distance measuring capabilities may be utilized to determine the position of the occupants 40, 41 in relation to each other with the slant angle controller steering the disinfecting curtain to separate the two occupants 40, 41. In some implementations a camera sensor or a microphone is used to determine if e.g. occupant 40 talks loudly or is coughing, in response the slant angle controller may adjust the slant angle so as to bring the disinfecting curtain 140 closer to occupant 40.

In some implementations, the longitudinal width of the disinfecting curtain 140 may be controlled by the slant angle controller selectively activating one or more light elements of the light source(s). For example, if only a first occupant 40 and a second occupant 41 are present and conversing with each other the disinfecting curtain 140 may extend with a first longitudinal width. If the presence of a third occupant 42 is detected together with the first occupant 40 (both facing the second occupant 41) the longitudinal width of the disinfecting light curtain 140 may be extended, e.g. by the slant angle controller selectively activating additional disinfecting light sources.

With reference to Fig. 3 there is depicted a disinfecting device 1 and disinfecting light being emitted in a first optical plane 140 with a first slant angle S and disinfecting light being emitted in a second optical plane 142 with a second slant angle S2. The slant angle S, S2 of the optical plane 140, 142 is depicted as measured from a normal N of the mounting surface 2 and placed at the beginning of the optical plane 140 at the lightbar 10 and mounting surface 2. The light bar 10 may be configured to selectively emit a disinfecting light curtain 140, 142 in one or both of the slant angles S and S2. In some embodiments, the light bar is configured to control the slant angle S, S2 of the disinfecting curtain 140, 142 such that approximately any slant angle S, S2 in the full + 90 to - 90 degree range (with respect to the normal N) is reachable.

In some implementations the disinfecting device is in communication with and/or comprises an integrated context awareness sensor 160. The context awareness sensor 160 may comprise an image sensor, IR sensor, thermophile sensor or audio sensor (e.g. a microphone) to sense a current context of the indoor space, e.g. whether or not one or more occupants are present in the indoor space. The context awareness sensor 160 may employ UWB radar LIDAR or ultrasound to actively (by means of both transmitting and receiving) detect the presence or movements of occupants in the indoor space 4. For example, an external context awareness sensor 160 may be in wireless communication with the control unit 120, over e.g. a Wi-Fi network. In some implementations the context awareness sensor 160 determines the position of the occupants relative the disinfecting device 1 and/or the position of the occupant inside the indoor space 4. Additionally, the context awareness sensor 160 may determine the orientation of an occupant and/or estimate the field of view of the occupant. It may also estimate probable movements or future positions of the occupants and adjust the disinfection light appropriately. Alternatively, the context awareness sensor may be a radiofrequency transceiver that is part of a radiofrequency-based sensing system configured to detect the position of the occupant inside the indoor space.

The control unit 120 may be configured to control the slant angle S, S2 of the disinfecting curtain 140, 142 such that the disinfecting curtain 140, 142 is at least a predetermined minimum distance (e.g. 30 cm) from the occupant or the eyes of the occupant. The control unit 120 may be configured to control slant angle S, S2 of the disinfecting curtain 140, 142 such that the disinfecting curtain 140, 142 is at most a predetermined maximum distance from the occupant. The slant angle may thus be controlled accordingly so as to not directly expose a moving occupant with disinfecting radiation (e.g. UV radiation).

The context awareness sensor 160 may detect a current context for the indoor space 4 and/or the control unit 120 may be manually configured with a fixed context for the indoor space 4. The indoor space context may indicate that the occupants are performing physical exercise and in response the disinfecting light curtain 140, 142 should be slanted so that the disinfecting light curtain 140, 142 is closer to the mouth of the occupants as more viruses or pathogens are expelled during physical exercise. The disinfecting device(s) 1 of the indoor space may be manually controlled via a user device being in communication with the controllers 120 of each disinfecting device 1 and/or a central control unit. The user may specify the settings, such as the slant angle S, S2 (and the intensity, extension along the longitudinal axis and/or the spectra of the outputted disinfecting light), of each disinfecting device 1 in the indoor space 4 via the user device. The user device presents the installed position of each disinfecting device 1 e.g. as an architectural map of the indoor space 4 or a scanned representation of the indoor space 4 acquired by the user device. The user may select each disinfecting device 1 and specify the settings of the disinfecting device 1 with the controller 120 of the disinfecting device 1 being configured to realize the specified settings.

The context awareness sensor 160 may also be informed about occupant's activities using a connection to information as provided by body worn sensing devices like mobile phones, fitness wrist bands or smartwatches. Alternatively, the context awareness sensor 160 may also be connected to information as provided by activity monitoring of the space e.g. training machinery being present in a gym, spot controls or microphones allowing to detect the active actor or musician on a theater stage. To this end, the body worn sensing devices, training machinery and microphones may convey sensor data to the context awareness sensor via a wireless network. For example, in response to the context awareness sensor establishing that a piece of gym machinery is unused the slant angle controller 120 may adjust the slant angle of the optical plane so as to sweep disinfecting light across the unused piece of gym machinery. In response to the context awareness sensor establishing that a piece of gym machinery is in use (either by detecting activity near the machinery or receiving an indication from the gym machinery indicating that it is in use), the slant angle controller 120 may adjust the slant angle such that the optical plane is placed in proximity to the user of the gym machinery. Accordingly, any pathogens expelled by the user of the gym machinery may be effectively disinfected by the disinfecting light. As gym and exercise activities are associated with rapid and heavy breathing which in turn may enable a greater spread of pathogens the context awareness sensor 160 may associate a gym machine being used with a potential increased risk of pathogen spread and adjust the slant angle, intensity of output disinfecting light spectrum accordingly.

The controller 120 may be configured to control the intensity (including the on/off state) of the disinfecting light curtain 140, 142. For example, the controller 120 may activate the disinfecting light curtain 140, 142 only in response to detecting two occupants in the indoor space which may be separated with the disinfecting light curtain. The context awareness sensor may detect that an occupant is talking loudly, sneezing or coughing and prompt the controller 120 to implement a disinfecting curtain 140, 142 with higher intensity and/or a disinfecting curtain 140, 142 slanted so as be closer to the occupant in order to more effectively prohibit spread of pathogens from the occupant.

In another example, the context awareness sensor 160 may recognize individual occupants (e.g. with a camera sensor and means of image recognition) in the indoor space 4 and assign each occupant with a personal disinfecting dose budget (e.g. a UV dose budget) which is used to control the slant angle S, S2 of the disinfecting curtain. The disinfecting dose budget may be linked to a personal schedule of the individual occupant, so as to e.g. keep an extra minimum distance from occupants with many personal meetings scheduled in the indoor space and keep a decreased minimum distance from occupants with few personal meetings scheduled in the indoor space. As a busy person with many personal meetings is expected to be in close proximity to active disinfecting curtains 140, 142 for a longer time the disinfecting light curtain 140, 142 may be slanted so as to be further away from the busy occupant or employ disinfecting light with lower intensity to keep the disinfecting exposure of the busy person below a recommended exposure threshold. On the other hand, for an occupant with few personal meetings the disinfecting dose budget may allow to employ disinfecting light curtains 140, 142 with higher intensity or control the slant angle of the disinfecting light curtains so as to be closer to the less busy occupant in order to minimize spread of pathogens while keeping the disinfecting exposure of the less busy occupant below a recommended exposure threshold. The disinfecting curtain generated by the disinfecting device 1 may lead to stray disinfecting light reaching an occupant due to the disinfecting light reflecting off objects such as floors or metal tables.

The context awareness sensor 160 may comprise a disinfecting light reflection sensor. The disinfecting reflection sensor being adapted to detect the intensity and/or spatial distribution of the reflected disinfecting light incident on the disinfecting reflections sensor. The disinfecting reflections sensor is configured to detect reflected disinfecting radiation in the same disinfecting spectrum as the spectrum emitted by the at least one disinfecting light source of the light bar 10. If no occupant breaches the disinfecting curtain 140, 142 the detected reflected disinfecting light may remain approximately constant (when the slant angle S, S2 is kept constant) or at least change in a predictable (e.g. prerecorded) fashion when the slant angle S, S2 is altered by the slant angle controller 120, accordingly, and the disinfecting light detected by the disinfecting reflection sensor may be identified to be in an undisturbed state. If an occupant breaches the disinfecting curtain 140, 142 the intensity and/or spatial incidence distribution of the disinfecting light incident on the reflection sensor changes in a sudden and/or unpredicted manner. By detecting such changes in the detected intensity and/or distribution of the reflected disinfecting light the controller may establish that an occupant is within a predetermined distance from the disinfecting curtain 140, 142 and in response adjust the slant angle or decrease the intensity of the disinfecting light outputted by the disinfecting light source (e.g. turn it off completely) so as to decrease the disinfecting light exposure of the occupant.

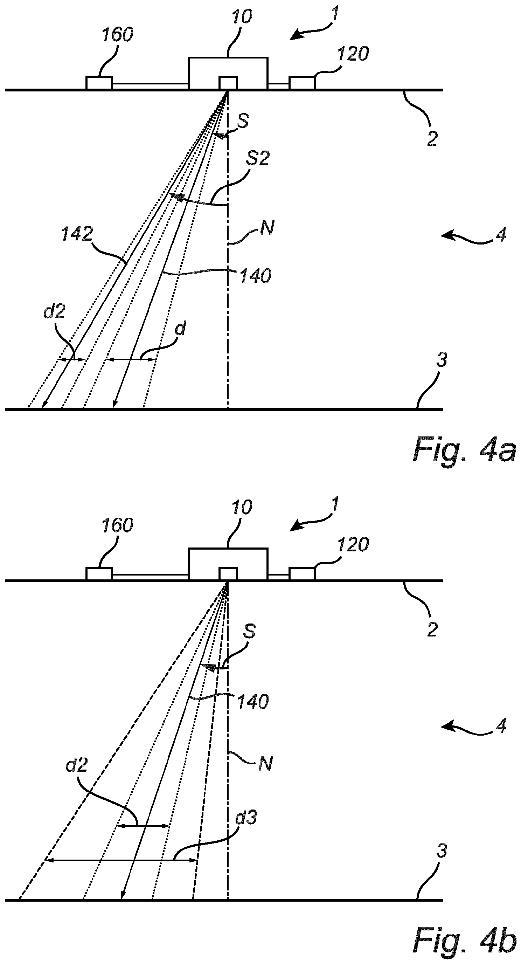

With reference to Fig. 4a there is depicted a light bar 10 with two potential disinfecting light curtains 140, 142 which the light bar 10 may emit simultaneously and/or separately (for example by changing the slant angle from S to S2). In general, the disinfecting light emitted by the light bar 10 may have some slant angle distribution, or slant width, d. In such cases, the slant angle S of the optical plane may be defined as the peak intensity slant angle S or the average slant angle S of the emitted disinfecting light (for example the average slant angle with respect to the disinfecting light intensity distribution). In some implementations the light bar 10 comprises two disinfecting light sources, the light sources being adapted to emit disinfecting light in two different wavelength bands. The light bar 10 is configured to output the disinfecting light of the second disinfecting light source in second optical plane 142 (as a second disinfecting light curtain) neighboring the optical plane 140 of the first disinfecting light source. That is, the slant angle S2 of the second optical is different from the slant angle of the first optical plane, e.g. the second optical plane 142 is arranged to form an angle of 10 degrees with the first optical plane 140 while both optical planes 140, 142 extend from the same longitudinal axis of the light bar 10. Moreover, a third disinfecting curtain may be emitted on the opposite side (with respect to the second disinfecting curtain 142) of the first optical plane 140 so as to surround a middle optical plane 140 a respective side optical plane 142. That is, e.g. a first disinfecting curtain 140 at slant angle S, a second disinfecting curtain 142 at slant angle S2 and a third disinfecting curtain at slant angle S + (S - S2). This may be referred to as a double disinfecting curtain or an enveloped curtain. With the inner disinfecting curtain 140 forming an inner envelope curtain and the second and third disinfecting curtain forming the outer envelope curtain.

With reference to Fig. 4b another form disinfecting double or envelope disinfecting curtain 140 is depicted. The light bar 10 may be configured to shape the disinfecting light of a second disinfecting light source so as to with a large slant angle width d3 envelope disinfecting light curtain of the first light source with a small slant angle width d2. The disinfecting light of the second light source envelopes the first disinfecting light in at least the slant angle direction. The second disinfecting light may form an outer envelope around the disinfecting light curtain 140 of the first light source. For example, the second disinfecting light emitted by the light bar may have a broad slant angle width d3 with the first disinfecting light emitted by the light bar having a narrow slant angle width d2 centered in the second disinfecting light distribution. The slant angle controller 120 may be configured to steer the slant angle of both the outer and inner disinfecting light such that their respective optical plane 140 remains the same.

Preferably, the second light source outputs disinfecting radiation which has higher maximum recommended dosage levels for humans compared to the disinfecting radiation of the first light source. For example, the first light source outputs disinfecting light in a band centered around 255 nm and the second light source outputs disinfecting light in a band centered around 222 nm. Alternatively, the second light source outputs visible blue light with 405 nm wavelength. While still being visible light the 405 nm blue light features some germicidal properties. Accordingly, the any occupant must first breach the second disinfecting curtain with slant width d3 prior to reaching the first disinfecting curtain with slant width d2.

In some, implementations the double disinfecting curtain is used together with a context awareness sensor 160 comprising a reflection sensor configured to detect reflected disinfecting radiation of at least the first light source (forming e.g. the outer envelope disinfecting curtain width slant width d3). If the controller 120 detects that an occupant has breached the first disinfecting curtain (e.g. by detecting a sufficient change in the detected reflected disinfecting radiation) the controller 120 may adjust the slant angle S so as to steer the first and second disinfecting curtain away from the occupant and/or disable the first and/or second disinfecting light source. In this way, less harmful light sources (e.g. IR and 405 nm) may form an outer envelope disinfecting light curtain doubling as a disinfection curtain and a presence detecting curtain (in combination with the disinfecting reflection sensor in the context awareness sensor 160) while more harmful but more efficient germicidal disinfecting light sources (e.g. employing UV-C) form an inner envelope disinfecting light and acts as a disinfecting curtain. If an occupant breaches the outer disinfecting curtain the inner disinfecting curtain may be disabled.

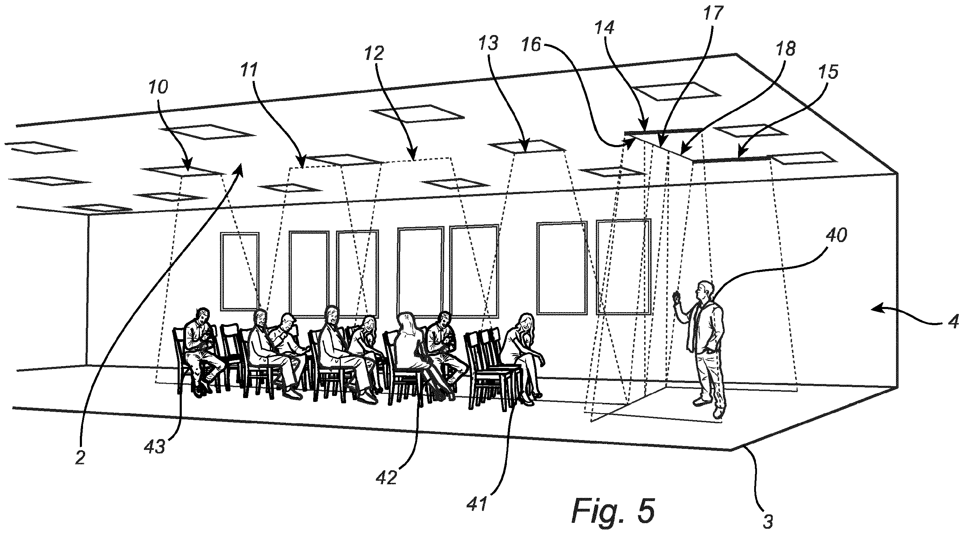

Fig. 5 depicts a system of disinfecting devices with respective light bars 10, 11, 12, 13, 14, 16, 17, 18 mounted to a surface 2 of an indoor space 4. The occupants 40, 41, 42, 43 of the indoor space 4 may be attending a lecture or presentation. One or more context awareness sensor may be provided in the indoor space 4 and convey information to a central control unit being in communication with the slant angle controller of each disinfecting device. For example, the context awareness sensor may detect that occupant 41 and 42 may be separated by emitting a disinfecting curtain from e.g. light bar 12 and 13. Accordingly, the central control unit instructs the slant angle controller of the relevant disinfecting devices such that light bar 12 and 13 emits a disinfecting light curtain at a respective slant angle suitable for separating occupant 41 and 42. The disinfecting curtains emitted by a system of disinfecting devices may be arranged so as to generate a continuous screen or bubble around an occupant 40, 41, 42. Accordingly, the continuous surrounding screen of disinfection curtains together with the surfaces of the indoor space may provide the occupant with a private air volume wherein all air reaching the private air volume is disinfected by at least one disinfecting curtain. For multiple occupants present in the indoor space each occupant may be provided with her/his own continuous screen of disinfection curtains.

The disinfecting devices and the respective light bars 10-18 may be arranged in a grid to be distributed in a sector of or the entire indoor space 4. For example, light bars 10-15 may be arranged such that the optical plane extends from a longitudinal axis while light bars 16-18 extends from a respective axis perpendicular to the longitudinal axis, a latitudinal axis. In this indoor space 4 such an arrangement of the light bars is suitable as a lecturing occupant 40 may be separated from the seated listening occupants 41-43 with one or more latitudinal disinfecting curtains emitted from light bars 16-18 while the seated occupants 41-43 are separated from each other with longitudinal disinfecting curtains from light bars 10-15. In accordance with some implementations a visible light source may be configured to emit visible light that indicates the position of the disinfecting light curtain. For example, the visible light may be projected with the disinfecting curtains emitted by the light bars 14-18 onto the floor 3 around the presenting occupant 40. The presenting occupant will thereby be aware of the presence of the disinfecting curtains and the extent of the assigned stage area which is isolated by disinfecting curtains. Similarly, a visible light source may indicate the current position of the disinfecting curtains emitted by light bars 10-13 making the occupants 41-43 aware of the presence of the disinfecting curtains and how close they may move to their neighboring occupant. The visible light sources may be provided externally and controlled by the central control unit to collectively indicate the position of the disinfecting light curtains in the indoor space.

A disinfection device or system of disinfection devices may similarly be used to mitigate spread of pathogens between stage performers on a stage and/or between a stage performer and an audience of the stage performer. While singing or speaking loudly a stage performer may expel more pathogens and spread the pathogens over a greater distance. To this end a context awareness sensor may be in communication with the microphones worn or used by the stage performers to establish that a stage performer is e.g. singing by receiving an indication from the microphone indicating that the microphone is active and/or recording audio exceeding a predetermined volume level. In response to the context awareness sensor establishing that a microphone is in use the slant angle controller may steer the slant angle of the optical plane close to the face of the stage performer so as to disinfect any pathogens expelled by the stage performer. The context awareness sensor may further determine the position of the stage performer on the stage and approach the edge of the stage when the stage performer approaches the edge of the stage.

Accordingly, the risk of stage performers infecting each other and/or the risk of a stage performer expelling pathogens into the audience is mitigated by the disinfection device.

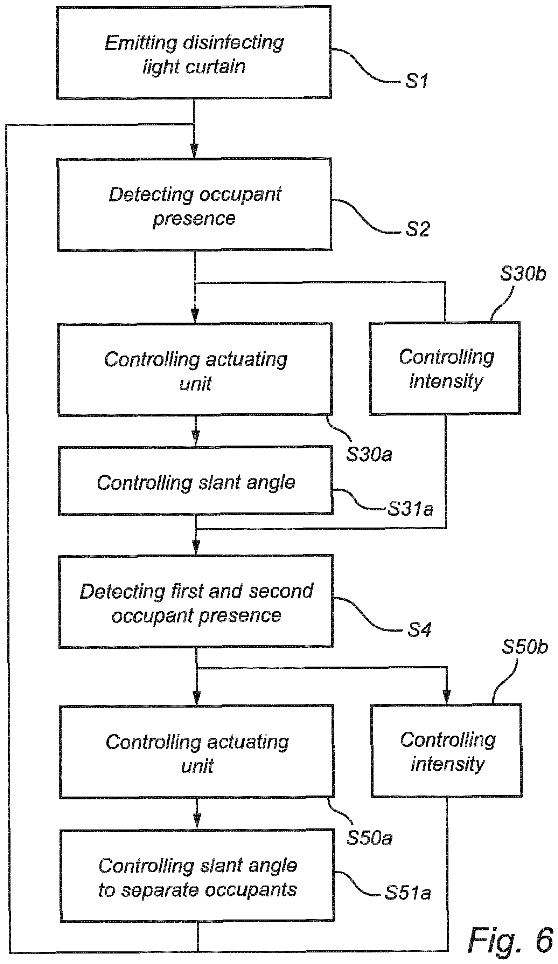

Fig. 6 is a flow chart describing a method according to some implementations of the invention. At S 1 disinfecting light is emitted from the light source of the light bar and shaped so as to extend in an optical plane. The emitted disinfecting light may be referred to as a disinfecting light curtain. At optional step S2 a context awareness sensor detects the presence of an occupant and communicates information representing the presence to the slant angle controller. In response to occupant presence being detected the method may to S31a comprising controlling the slant angle of the light bar. Optionally, controlling the slant angle of the light bar is enabled by controlling an actuating unit at S30a which steers the orientation and/or position of at least one of the light bar, the light source(s) of the light bar and the optical element of the light bar. In some implementations, the slant angel controller is further configured to control the intensity of the emitted disinfecting light shaped by the optical element. Thus, the method may additionally perform step S30b and control the intensity of the emitted disinfecting light. For example, if occupant presence is detected the intensity is increased. The above mentioned steps may be repeated, for example the context awareness sensor collects an updated data reading which changes the occupant presence state of the indoor space or the control of the slant angle by the slant angle controller is scheduled to assume certain angles at certain periods of time.

For example, the method may at S4 detect the presence of a first and a second occupant and in response control the slant angle to separate the two occupants at S51a. Optionally, controlling the slant angle may include controlling an actuator at S50a. In some implementations, the light bar is configured to output disinfecting light in at least one out of two optical planes. With such a light bar the slant angle controller may selectively control the light bar to emit disinfecting light extending along the optical plane which separates the two occupants. The slant angle controller may optionally control the intensity of the disinfecting light outputted by the light bar at S50b. That is, if e.g. the emitted disinfecting light separates two occupants the intensity may be increased.

The context awareness sensor may determine more detailed presence information regarding the occupants, for example whether the two occupants are facing each other or not. In response to the two occupants facing each other a slant angle separating the two occupants is assigned, e.g. with increased intensity to enhance disinfection. If the occupants are not facing each other the disinfecting curtain may not necessarily be controlled to separate the occupants, e.g. with a decreased intensity, to enhance the power efficiency and the lifetime of disinfecting device.

The person skilled in the art realizes that the present invention by no means is limited to the preferred embodiments described above. On the contrary, many modifications and variations are possible within the scope of the appended claims. For example, the light bar may comprise a plurality of different light sources outputting light in different disinfecting spectra wherein the slant angle controller may selectively activate one or more of the light sources outputting different disinfecting spectra so as to not only control the slant angle optionally one of the longitudinal width and the intensity, but also the type of disinfecting radiation emitted.

## Claims

1. A disinfecting device (1) comprising:
an elongated light bar (10) configured to be mounted to a surface (2) of an indoor space (4), said elongated light bar (10) comprising
at least one light source (100), configured to emit disinfecting light for disinfecting pathogens, the disinfecting device (1) being **characterized in that** it further comprises:
at least one optical element (102), configured to shape disinfecting light emitted by the at least one light source (100), such that the elongated light bar (10) is configured to emit a disinfecting light curtain substantially confined in an optical plane (140) extending from a longitudinal axis of the elongated bar (10); and
a slant angle controller (120), configured to, when the disinfecting device (1) is mounted to the surface (2), control a slant angle (S) between the optical plane (140) and a normal (N) of the surface (4).

2. The disinfecting device (1) according to claim 1, wherein said at least one light source is configured to emit at least one of ultraviolet light and visible light with a maximum intensity for a wavelength between 400 and 410 nm.

3. The disinfecting device (1) according to claim 1 or 2, wherein the slant angle controller (120) is configured to control the positioning of the at least one optical element (102) with respect to the at least one light source (100).

4. The disinfecting device (1) according to any of the preceding claims, wherein the disinfecting device (1) comprises an actuating unit and said slant angle controller (120) is configured to control the actuating unit,
wherein, when said disinfecting device (1) is mounted to a surface (4), said actuating unit is configured to steer the orientation of the elongated bar (10) with respect to the surface (4).

5. The disinfecting device (1) according to any of the preceding claims, wherein
the elongated light bar (10) is configured to emit the disinfecting light curtain in two optical planes (140, 142), wherein the slant angle (S, S2) of the two optical planes (140, 142) is different, and
wherein the slant angle controller (120) is configured to selectively control the elongated light bar (10) to selectively emit light in one of said optical planes (140, 142).

6. The disinfecting device (1) according to any of the preceding claims, wherein the slant angle controller (120) is configured to communicate with a context awareness sensor (160) and control the slant angle (S) of the optical plane based on an occupant (40, 41, 42, 43) context being detected by the context awareness sensor (160).

7. The disinfecting device according to claim 5, wherein said context awareness sensor (160) is configured to detect the position of an occupant (40, 41, 42, 43) relative the disinfecting device (1) and wherein the slant angle controller (120) is configured to control the slant angle (S) of the optical plane (140) based on the position and/or activity of the occupant (40, 41, 42, 43).

8. The disinfecting device (1) according to any of the preceding claims, wherein said at least one light source (100) comprises a plurality of light elements distributed longitudinally along the elongated light bar (10) and the slant angle controller (120) is configured to activate a subset of the light elements to control an extent of the disinfecting light curtain (140) along the longitudinal axis.

9. The disinfecting device (1) according to any of the preceding claims, further comprising a visible spectrum light source (101) configured to project visible light, the projected visible light indicating the position of the optical plane (140).

10. The disinfecting device (1) according to any of the preceding claims, wherein the elongated light bar (10) is further configured to emit an enveloping light, the enveloping light surrounding the disinfecting light emitted in the optical plane (140) and having a different spectrum.

11. The disinfecting device (1) according to any of the preceding claims, wherein the disinfecting light curtain is substantially confined in the optical plane with a tolerance of at most 2 centimeter offset from the optical plane.

12. The disinfecting device (1) according to claim 11, wherein the slant angle controller (120) is configured to communicate with a reflection sensor configured to detect light from the at least one light source (100) reflected from the indoor space (4), and wherein the slant angle (S) of the optical plane is controlled in response to a deviation exceeding a predetermined threshold value in the sensed reflected light being detected by the reflection sensor.

13. A disinfecting system comprising a plurality of disinfecting devices (1) according to any of preceding claims and a central control unit, wherein the central control unit is configured to control the slant angle controller (120) of each disinfecting device (1).

14. A method for controlling a disinfecting device (1) according to any of the preceding claims, the disinfecting device (1) comprising an elongated light bar (10) configured to be mounted to a surface (2) of an indoor space (4), said light bar (10) comprising at least one light source (100) configured to emit disinfecting light for disinfecting pathogens, and at least one optical element (102) configured to shape disinfecting light emitted by the at least one light source (100), the method being **characterized in that** it comprises the steps of:
emitting a disinfecting light curtain (step S1) substantially confined in an optical plane (140) extending from a longitudinal axis of the light bar (10); and
controlling, when the disinfecting device (1) is mounted to the surface (2), a slant angle (S) between the optical plane (140) and a normal (N) of the surface (4).

15. The method according to claim 14 further comprising the steps of:
determining the position of a first occupant (40) and a second occupant (41) in the indoor space (4) relative the disinfecting device (step S4); and
controlling the slant angle of the light curtain so as to separate said first occupant (40) from said second occupant (41) with the disinfecting curtain (step S51a).

## Patentansprüche

1. Desinfektionsvorrichtung (1), umfassend:
eine längliche Lichtleiste (10), die konfiguriert ist, um an einer Oberfläche (2) eines Innenraums (4) montiert zu werden, die längliche Lichtleiste (10) umfassend:
mindestens eine Lichtquelle (100), die konfiguriert ist, um Desinfektionslicht zum Desinfizieren von Krankheitserregern zu emittieren, wobei die Desinfektionsvorrichtung (1) **dadurch gekennzeichnet ist, dass** sie ferner umfasst:
mindestens ein optisches Element (102), das konfiguriert ist, um Desinfektionslicht, das durch die mindestens einen Lichtquelle (100) EMITTIERT WIRD, DERART zu FORMEN, dass die längliche Lichtleiste (10) konfiguriert ist, um einen Desinfektionslichtvorhang zu emittieren, der im Wesentlichen in einer optischen Ebene (140) begrenzt ist, die sich von einer Längsachse der länglichen Leiste (10) aus erstreckt; und
eine Neigungswinkelsteuerung (120), die konfiguriert ist, um wenn Desinfektionsvorrichtung (1) an die Oberfläche (2) montiert ist, einen Neigungswinkel (S) zwischen der optischen Ebene (140) und einer Normalen (N) der Oberfläche (4) zu steuern.

2. Desinfektionsvorrichtung (1) nach Anspruch 1, wobei die mindestens eine Lichtquelle konfiguriert ist, um mindestens eines von ultraviolettem Licht und sichtbarem Licht mit einer maximalen Intensität für eine Wellenlänge zwischen 400 und 410 nm zu emittieren.

3. Desinfektionsvorrichtung (1) nach Anspruch 1 oder 2, wobei die Neigungswinkelsteurung (120) konfiguriert ist, um die Positionierung des mindestens einen optischen Elements (102) hinsichtlich der mindestens einen Lichtquelle (100) zu steuern.

4. Desinfektionsvorrichtung (1) nach einem der vorstehenden Ansprüche, wobei die Desinfektionsvorrichtung (1) eine Betätigungseinheit umfasst und die Neigungswinkelsteuerung (120) konfiguriert ist, um die Betätigungseinheit zu steuern,
wobei, wenn die Desinfektionsvorrichtung (1) an einer Oberfläche (4) montiert ist, die Betätigungseinheit konfiguriert ist, um die Ausrichtung der länglichen Leiste (10) hinsichtlich der Oberfläche (4) zu steuern.

5. Desinfektionsvorrichtung (1) nach einem der vorstehenden Ansprüche, wobei die längliche Lichtleiste (10) konfiguriert ist, um den Desinfektionslichtvorhang in zwei optischen Ebenen (140, 142) zu emittieren, wobei der Neigungswinkel (S, S2) der zwei optischen Ebenen (140, 142) unterschiedlich ist, und
wobei die Neigungswinkelsteuerung (120) konfiguriert ist, um die längliche Lichtleiste (10) selektiv zu steuern, um Licht in einer der optischen Ebenen (140, 142) selektiv zu emittieren.

6. Desinfektionsvorrichtung (1) nach einem der vorstehenden Ansprüche, wobei die Neigungswinkelsteuerung (120) konfiguriert ist, um mit einem Kontextsensitivitätssensor (160) zu kommunizieren und den Neigungswinkel (S) der optischen Ebene basierend auf einem Kontext eines Nutzers (40, 41, 42, 43) zu steuern, der durch den Kontextsensitivitätssensor (160) erkannt wird.

7. Desinfektionsvorrichtung nach Anspruch 5, wobei der Kontextsensitivitätssensor (160) konfiguriert ist, um die Position eines Nutzers (40, 41, 42, 43) relativ zu der Desinfektionsvorrichtung (1) zu erkennen, und wobei die Neigungswinkelsteuerung (120) konfiguriert ist, um den Neigungswinkel (S) der optischen Ebene (140) basierend auf der Position und/oder der Aktivität des Nutzers (40, 41, 42, 43) zu steuern.

8. Desinfektionsvorrichtung (1) nach einem der vorstehenden Ansprüche, wobei die mindestens eine Lichtquelle (100) eine Vielzahl von Lichtelementen umfasst, die in Längsrichtung entlang der länglichen Lichtleiste (10) verteilt ist, und wobei die Neigungswinkelsteuerung (120) konfiguriert ist, um eine Untermenge der Lichtelemente zu aktivieren, um eine Ausdehnung des Desinfektionslichtvorhangs (140) entlang der Längsachse zu steuern.

9. Desinfektionsvorrichtung (1) nach einem der vorstehenden Ansprüche, ferner umfassend eine Lichtquelle (101) für ein sichtbares Spektrum, die konfiguriert ist, um sichtbares Licht zu projizieren, wobei das projizierte sichtbare Licht die Position der optischen Ebene (140) anzeigt.

10. Desinfektionsvorrichtung (1) nach einem der vorstehenden Ansprüche, wobei die längliche Lichtleiste (10) ferner konfiguriert ist, um ein Hülllicht zu emittieren, wobei das Hülllicht das Desinfektionslicht umgibt, das in der optischen Ebene (140) emittiert wird und ein unterschiedliches Spektrum aufweist.

11. Desinfektionsvorrichtung (1) nach einem der vorstehenden Ansprüche, wobei der Desinfektionslichtvorhang im Wesentlichen auf die optische Ebene beschränkt ist, mit einer Toleranz von höchstens 2 Zentimetern Versatz von der optischen Ebene.

12. Desinfektionsvorrichtung (1) nach Anspruch 11, wobei die Neigungswinkelsteuerung (120) konfiguriert ist, um mit einem Reflexionssensor zu kommunizieren, der konfiguriert ist, um Licht von der mindestens einen Lichtquelle (100) zu erkennen, das von dem Innenraum (4) reflektiert wird, und wobei der Neigungswinkel (S) der optischen Ebene als Reaktion auf eine Abweichung gesteuert wird, die einen zuvor bestimmten Schwellenwert in dem erfassten reflektierten Licht überschreitet, das durch den Reflexionssensor erkannt wird.

13. Desinfektionssystem, umfassend eine Vielzahl von Desinfektionsvorrichtungen (1) nach einem der vorstehenden Ansprüche und eine zentrale Steuereinheit, wobei die zentrale Steuereinheit konfiguriert ist, um die Neigungswinkelsteuerung (120) jeder Desinfektionsvorrichtung (1) zu steuern.

14. Verfahren zum Steuern einer Desinfektionsvorrichtung (1) nach einem der vorstehenden Ansprüche, die Desinfektionsvorrichtung (1) umfassend eine längliche Lichtleiste (10), die konfiguriert ist, um an einer Oberfläche (2) eines Innenraums (4) montiert zu werden, die Lichtleiste (10) umfassend mindestens eine Lichtquelle (100), die konfiguriert ist, um Desinfektionslicht zum Desinfizieren von Krankheitserregern zu emittieren, und mindestens ein optisches Element (102), das konfiguriert ist, um Desinfektionslicht zu formen, das durch die mindestens eine Lichtquelle (100) emittiert wird, wobei das Verfahren **dadurch gekennzeichnet ist, dass** es die Schritte umfasst:
Emittieren eines Desinfektionslichtvorhangs (Schritt S1), der im Wesentlichen auf eine optische Ebene (140) begrenzt ist, die sich von einer Längsachse der Lichtleiste (10) aus erstreckt;
und Steuern, wenn die Desinfektionsvorrichtung (1) an der Oberfläche (2) montiert ist, eines Neigungswinkels (S) zwischen der optischen Ebene (140) und einer Normalen (N) der Oberfläche (4).

15. Verfahren nach Anspruch 14, ferner umfassend die Schritte:
Bestimmen der Position eines ersten Nutzers (40) und eines zweiten Nutzers (41) in dem Innenraum (4) relativ zu der Desinfektionsvorrichtung (Schritt S4); und
Steuern des Neigungswinkels des Lichtvorhangs, um den ersten Nutzer (40) von dem zweiten Nutzer (41) durch den Desinfektionsvorhang zu trennen (Schritt S51a).

## Revendications

1. Dispositif de désinfection (1) comprenant :
une barre de lumière allongée (10) conçue pour être montée sur une surface (2) d'un espace intérieur (4), ladite barre de lumière allongée (10) comprenant :
au moins une source de lumière (100), configurée pour émettre une lumière de désinfection pour désinfecter des agents pathogènes, le dispositif de désinfection (1) étant **caractérisé en ce qu'**il comprend en outre :
au moins un élément optique (102), configuré pour façonner une lumière de désinfection émise par l'au moins une source de lumière (100), de sorte que la barre de lumière allongée (10) est configurée pour émettre un rideau de lumière de désinfection sensiblement confiné dans un plan optique (140) s'étendant à partir d'un axe longitudinal de la barre allongée (10) ; et
un dispositif de commande d'angle d'inclinaison (120), configuré pour, lorsque le dispositif de désinfection (1) est monté sur la surface (2), commander un angle d'inclinaison (S) entre le plan optique (140) et une normale (N) de la surface (4).

2. Dispositif de désinfection (1) selon la revendication 1, dans lequel ladite au moins une source de lumière est configurée pour émettre au moins une lumière ultraviolette et une lumière visible avec une intensité maximale pour une longueur d'onde comprise entre 400 et 410 nm.

3. Dispositif de désinfection (1) selon la revendication 1 ou 2, dans lequel le dispositif de commande d'angle d'inclinaison (120) est configuré pour commander le positionnement de l'au moins un élément optique (102) par rapport à l'au moins une source de lumière (100).

4. Dispositif de désinfection (1) selon l'une quelconque des revendications précédentes, dans lequel le dispositif de désinfection (1) comprend une unité d'actionnement et ledit dispositif de commande d'angle d'inclinaison (120) est configuré pour commander l'unité d'actionnement,
dans lequel, lorsque ledit dispositif de désinfection (1) est monté sur une surface (4), ladite unité d'actionnement est conçue pour diriger l'orientation de la barre allongée (10) par rapport à la surface (4).

5. Dispositif de désinfection (1) selon l'une quelconque des revendications précédentes, dans lequel la barre de lumière allongée (10) est configurée pour émettre le rideau de lumière de désinfection dans deux plans optiques (140, 142), dans lequel l'angle d'inclinaison (S, S2) des deux plans optiques (140, 142) est différent, et
dans lequel le dispositif de commande d'angle d'inclinaison (120) est configuré pour commander sélectivement la barre de lumière allongée (10) afin qu'elle émette sélectivement de la lumière dans l'un desdits plans optiques (140, 142).

6. Dispositif de désinfection (1) selon l'une quelconque des revendications précédentes, dans lequel le dispositif de commande d'angle d'inclinaison (120) est configuré pour communiquer avec un capteur sensible au contexte (160) et commander l'angle d'inclinaison (S) du plan optique en fonction du contexte d'un occupant (40, 41, 42, 43) détecté par le capteur sensible au contexte (160).

7. Dispositif de désinfection selon la revendication 5, dans lequel ledit capteur sensible au contexte (160) est configuré pour détecter la position d'un occupant (40, 41, 42, 43) par rapport au dispositif de désinfection (1) et dans lequel le dispositif de commande d'angle d'inclinaison (120) est configuré pour commander l'angle d'inclinaison (S) du plan optique (140) en fonction de la position et/ou de l'activité de l'occupant (40, 41, 42, 43).

8. Dispositif de désinfection (1) selon l'une quelconque des revendications précédentes, dans lequel ladite au moins une source de lumière (100) comprend une pluralité d'éléments de lumière répartis longitudinalement le long de la barre de lumière allongée (10) et le dispositif de commande d'angle d'inclinaison (120) est configuré pour activer un sous-ensemble des éléments de lumière afin de commander une étendue du rideau de lumière de désinfection (140) le long de l'axe longitudinal.

9. Dispositif de désinfection (1) selon l'une quelconque des revendications précédentes, comprenant en outre une source de lumière à spectre visible (101) configurée pour projeter une lumière visible, la lumière visible projetée indiquant la position du plan optique (140).

10. Dispositif de désinfection (1) selon l'une quelconque des revendications précédentes, dans lequel la barre de lumière allongée (10) est en outre configurée pour émettre une lumière enveloppante, la lumière enveloppante entourant la lumière de désinfection émise dans le plan optique (140) et ayant un spectre différent.

11. Dispositif de désinfection (1) selon l'une quelconque des revendications précédentes, dans lequel le rideau de lumière de désinfection est sensiblement confiné dans le plan optique avec une tolérance d'au plus 2 centimètres de décalage par rapport au plan optique.

12. Dispositif de désinfection (1) selon la revendication 11, dans lequel le dispositif de commande d'angle d'inclinaison (120) est configuré pour communiquer avec un capteur de réflexion configuré pour détecter une lumière provenant de l'au moins une source de lumière (100) réfléchie depuis l'espace intérieur (4), et dans lequel l'angle d'inclinaison (S) du plan optique est commandé en réponse à un écart dépassant une valeur seuil prédéterminée dans la lumière réfléchie détectée étant captée par le capteur de réflexion.

13. Système de désinfection comprenant une pluralité de dispositifs de désinfection (1) selon l'une quelconque des revendications précédentes et une unité de commande centrale, dans lequel l'unité de commande centrale est configurée pour commander le dispositif de commande d'angle d'inclinaison (120) de chaque dispositif de désinfection (1).

14. Procédé de commande d'un dispositif de désinfection (1) selon l'une quelconque des revendications précédentes, le dispositif de désinfection (1) comprenant une barre de lumière allongée (10) conçue pour être montée sur une surface (2) d'un espace intérieur (4), ladite barre de lumière (10) comprenant au moins une source de lumière (100) configurée pour émettre une lumière de désinfection pour désinfecter des agents pathogènes, et au moins un élément optique (102) configuré pour façonner une lumière de désinfection émise par l'au moins une source de lumière (100), le procédé étant **caractérisé en ce qu'**il comprend les étapes consistant à :
émettre un rideau de lumière de désinfection (étape S1) sensiblement confiné dans un plan optique (140) s'étendant à partir d'un axe longitudinal de la barre de lumière (10) ;
et commander, lorsque le dispositif de désinfection (1) est monté sur la surface (2), un angle d'inclinaison (S) entre le plan optique (140) et une normale (N) de la surface (4).

15. Procédé selon la revendication 14, comprenant en outre l'étape consistant à :
déterminer la position d'un premier occupant (40) et d'un second occupant (41) dans l'espace intérieur (4) par rapport au dispositif de désinfection (étape S4) ; et
commander l'angle d'inclinaison du rideau de lumière de manière à séparer ledit premier occupant (40) dudit second occupant (41) avec le rideau de désinfection (étape S51a).
